# FASCICULE DE BREVET EUROPEEN

(11) **EP 3 427 658 B1**
(45) Date de publication et mention de la délivrance du brevet: **28.10.2020**
(21) Numéro de dépôt: 18182592.8
(22) Date de dépôt: 10.07.2018
(51) Int. Cl.: A61B 5/04, A61B 5/0478, H03F 1/22

(54) **ELECTRODE DE MESURE D'ACTIVITÉ ÉLECTRIQUE IMPLANTABLE OU NON-IMPLANTABLE**
IMPLANTIERBARE ODER NICHT-IMPLANTIERBARE ELEKTRODE ZUM MESSEN DER ELEKTRISCHEN AKTIVITÄT
IMPLANTABLE OR NON-IMPLANTABLE ELECTRICAL ACTIVITY MEASURING ELECTRODE

(30) Priorité: 12.07.2017 FR 1756615
(43) Date de publication de la demande: 16.01.2019
(73) Titulaire: Commissariat à l'Énergie Atomique et aux Énergies Alternatives, 75015 Paris (FR)
(72) Inventeur: BECHE, Jean-François, 38500 COUBLEVIE (FR)
(74) Mandataire: INNOV-GROUP

(56) Documents cités:
- GB-A- 1 442 296
- US-A1- 2012 257 339
- US-A1- 2013 204 154

## Description

### Domaine technique de l'invention

La présente invention se rapporte au domaine des électrodes dites actives, permettant de mesurer une activité électrique à l'extérieur ou à l'intérieur du corps humain.

### Etat de la technique

Dans le domaine de la mesure d'une activité électrique dans un corps humain, il est connu de positionner un certain nombre d'électrodes sur ou dans le corps d'un patient pour enregistrer l'activité électrique. Il existe notamment les principes de mesure connus de type électroencéphalogramme (EEG) pour mesurer l'activité électrique du cerveau, électrocardiogramme (ECG) pour mesurer la performance du cœur ou électromyogramme (EMG) pour mesurer l'activité neuromusculaire. D'autres principes, notamment implantés dans le corps, existent également.

Parmi ces trois principes cités ci-dessus, le plus difficile à mettre en place est souvent l'électroencéphalogramme. Dans le cas d'une mesure de type EEG (pour Electroencephalographie), les électrodes sont placées sur la tête du patient pour mesurer l'activité électrique du cerveau. Les signaux prélevés sont alors très faibles et nécessitent une amplification.

L'architecture typique d'un système EEG se compose de plusieurs électrodes, souvent associées par paire, pour mesurer des différences de potentiel. Il est possible de mettre en place un montage dit "unipolaire" dans lequel on utilise une électrode active et une électrode de référence ou un montage dit "bipolaire" dans lequel on utilise deux électrodes actives. Dans un montage unipolaire, l'électrode de référence est positionnée dans un endroit à faible activité électrique.

Comme décrit ci-dessus, en EEG, la différence de potentiel mesurée entre deux électrodes est très faible, de l'ordre du millivolt ou même du microvolt. De plus, les électrodes EEG ne captent pas seulement le signal électrique provenant du cerveau. Les électrodes sont extrêmement sensibles à leur environnement ; elles peuvent capter le bruit électromagnétique généré par des appareils électriques environnants (p. ex., ordinateur, téléphone cellulaire, amplificateur, éclairage).

Pour améliorer la détection, il existe plusieurs solutions. L'une d'elles consiste à améliorer la conductivité entre l'électrode et la peau du patient, par exemple en appliquant un gel. Cependant, il est désormais intéressant d'utiliser des électrodes dites "sèches".

Une autre solution consiste bien entendu à amplifier le signal mesuré entre les deux électrodes. Pour cela, il a notamment été proposé de relier chaque électrode à un boîtier d'amplification déporté, à travers des câbles de liaison. Le boîtier d'amplification comporte notamment une solution d'amplification, un système de filtrage et un convertisseur analogique/numérique.

Cependant, les câbles de liaison étant particulièrement longs, ils ont tendance à :
- Jouer le rôle d'antenne, captant alors le bruit de fond environnant et venant perturber le signal obtenu au niveau des électrodes ;
- Entraîner une dégradation du signal, due à l'impédance du câble qui se rajoute à celle de l'interface corps-électrode ;

Pour éviter ces désagréments, il a été proposé d'intégrer une électronique dans le boîtier de l'électrode. Cette électronique inclut par exemple un préamplificateur de manière à amplifier le signal dès sa sortie. Il est ensuite de nouveau amplifié dans un boîtier central déporté. Mais, dans ces solutions, même si le signal est déjà amplifié au niveau de l'électrode, c'est-à-dire au plus près de la zone de détection, un câble la relie au boîtier central d'amplification, ce qui est toujours susceptible de venir perturber le signal mesuré. De plus, le préamplificateur nécessite tout de même une source d'alimentation dans le boîtier de l'électrode, rendant cette solution peu adaptée à une implantation dans le corps humain.

La demande de brevet US2005/0215916A1 propose une solution alternative qui consiste à intégrer dans le boitier de chaque électrode une électronique comprenant un convertisseur analogique/numérique et de n'employer qu'un seul câble reliant l'ensemble des électrodes à un microcontrôleur. Un multiplexeur permet de sélectionner chaque électrode de manière individuelle pour récupérer chaque signal capté par les électrodes. Cette solution est cependant complexe à mettre en œuvre.

Une électrode active a également été décrite dans la demande de brevet GB1442296A**.** Cette électrode est réalisée sur la base d'un montage à drain commun. L'entrée est appliquée sur la grille d'un transistor à effet de champ, la sortie est disponible sur la source du transistor et son drain est connecté directement à une alimentation. Un tel montage est classique car il permet d'obtenir une forte impédance d'entrée et une faible impédance de sortie. Cependant, il n'est pas adapté pour résoudre les problèmes posés ci-dessus.Il en ressort que la plupart des solutions connues ne sont pas forcément satisfaisantes, car trop complexe, trop coûteuse ou pas toujours adaptées, notamment lorsqu'elles comportent une source d'alimentation dans le boîtier de l'électrode.

Le but de l'invention est donc de proposer une électrode active permettant de pallier les inconvénients des électrodes de l'état de la technique.

### Exposé de l'invention

Ce but est atteint par une électrode active de mesure d'activité électrique implantable ou non-implantable, qui comporte :
- Un boîtier ;
- Un capteur de mesure adapté sur ledit premier boîtier et destiné à capter un signal électrique d'entrée ;
- Ledit boîtier logeant un circuit électronique comprenant un transistor qui comporte une première borne, une deuxième borne reliée audit capteur et formant une entrée une troisième borne ;
- Un dispositif de connexion électrique comprenant un premier point de connexion relié à ladite première borne du transistor, un deuxième point de connexion relié à la troisième borne du transistor et un troisième point de connexion relié uniquement à la deuxième borne du transistor.

Par rapport au document GB1442296A cité précédemment, l'électrode active de l'invention présente la particularité de ne pas intégrer dans son boîtier le montage du transistor (qui est à source commune ou à émetteur commun). Ce montage est en effet matérialisé lors de la connexion de l'électrode avec l'unité électronique déportée décrite ci-dessous. Autrement dit, les trois points de connexion du dispositif de connexion électrique de l'électrode active sont connectés chacun directement à une seule borne du transistor et ne sont pas reliés entre eux pour fournir directement un signal de sortie au niveau de l'électrode.

Selon une réalisation particulière, le circuit électronique comporte un condensateur connecté au capteur et à la deuxième borne du transistor.

Selon une réalisation particulière, le circuit électronique comporte un dispositif de protection connecté au capteur et à la deuxième borne du transistor.

Selon une réalisation particulière, le transistor est choisi parmi un type bipolaire ou à effet de champ.

L'invention concerne également un dispositif de mesure d'activité électrique.

Dans une première configuration, le dispositif de mesure d'activité électrique, comporte :
- Une électrode active telle que définie ci-dessus,
- Une unité électronique déportée comprenant un deuxième boîtier logeant un circuit électronique qui comporte :
   ∘ Un transistor comprenant une première borne, une deuxième borne et une troisième borne ;
   ∘ Une première source de courant connectée à la troisième borne du transistor ;
   ∘ Une deuxième source de courant connectée à la première borne du transistor ;
   ∘ Une borne de sortie connectée à la première borne du transistor ;
   ∘ Une boucle de retour connectée à la borne de sortie et comprenant au moins une résistance et un condensateur connecté en parallèle de ladite résistance ;
- Ladite unité électronique déportée comprenant un dispositif de connexion électrique comprenant :
   ∘ Un premier point de connexion relié à la troisième borne du transistor ;
   ∘ Un deuxième point de connexion relié à la masse ;
   ∘ Un troisième point de connexion auquel est reliée la boucle de retour ;
- Un câble reliant l'électrode active à l'unité électronique déportée de sorte que :
   ∘ Le premier point de connexion du dispositif de connexion de l'électrode active est relié au premier point de connexion du dispositif de connexion de l'unité électronique déportée ;
   ∘ Le deuxième point de connexion du dispositif de connexion de l'électrode active est relié au deuxième point de connexion du dispositif de connexion de l'unité électronique déportée ;
   ∘ Le troisième point de connexion du dispositif de connexion de l'électrode active est relié au troisième point de connexion du dispositif de connexion de l'unité électronique déportée ;

Selon une réalisation particulière, le circuit électronique de l'électrode active peut comporter l'ensemble comprenant au moins une résistance et un condensateur connecté en parallèle de ladite résistance, connecté d'une part à la deuxième borne du transistor et d'autre part au troisième point de connexion. Dans cette configuration, le dispositif de mesure d'activité électrique comporte alors :
- Une électrode active intégrant l'ensemble formé de la résistance et du condensateur,
- Une unité électronique déportée comprenant un deuxième boîtier logeant un circuit électronique qui comporte :
   ∘ Un transistor comprenant une première borne, une deuxième borne et une troisième borne ;
   ∘ Une première source de courant connectée à la troisième borne du transistor ;
   ∘ Une deuxième source de courant connectée à la première borne du transistor ;
   ∘ Une borne de sortie connectée à la première borne du transistor ;
- Ladite unité électronique déportée comprenant un dispositif de connexion électrique comprenant :
   ∘ Un premier point de connexion relié à la troisième borne du transistor ;
   ∘ Un deuxième point de connexion relié à la masse ;
   ∘ Un troisième point de connexion relié à la borne de sortie ;
- Un câble reliant l'électrode active à l'unité électronique déportée de sorte que :
   ∘ Le premier point de connexion du dispositif de connexion de l'électrode active est relié au premier point de connexion du dispositif de connexion de l'unité électronique déportée ;
   ∘ Le deuxième point de connexion du dispositif de connexion de l'électrode active est relié au deuxième point de connexion du dispositif de connexion de l'unité électronique déportée ;
   ∘ Le troisième point de connexion du dispositif de connexion de l'électrode active est relié au troisième point de connexion du dispositif de connexion de l'unité électronique déportée ;

Selon une réalisation particulière, chaque transistor est choisi parmi un type bipolaire ou à effet de champ.

### Brève description des figures

D'autres caractéristiques et avantages vont apparaître dans la description détaillée qui suit, faite en liaison avec les figures annexées listées ci-dessous :
- La figure 1 représente de manière schématique, l'architecture d'un dispositif de mesure d'une activité électrique sur un être vivant, conforme à l'invention ;
- Les figures 2A et 2B représentent l'architecture électronique du dispositif de mesure d'une activité électrique sur un être vivant, conforme à l'invention, selon deux modes de réalisation distincts ;
- La figure 3 représente différentes architectures possibles de traitement du signal d'entrée ;

### Description détaillée d'au moins un mode de réalisation

De manière classique, comme décrit ci-dessus, l'enregistrement d'une activité électrique sur un corps humain est réalisé en utilisant plusieurs électrodes. Pour mesurer une activité électrique, il faut au moins deux électrodes. Il est possible de réaliser deux types de montage :
- Un montage dit unipolaire dans lequel on utilise une électrode de référence et une électrode active. Il s'agit alors de mesurer la différence de potentiel entre l'électrode active et l'électrode de référence ;
- Un montage dit bipolaire dans lequel on utilise deux électrodes actives. La différence de potentiel est alors mesurée entre ces deux électrodes ;

Une électrode est dite active lorsqu'elle est destinée à capter le signal souhaité.

Un système de mesure à plusieurs électrodes, par exemple employé pour un électroencéphalogramme (EEG), utilise donc plusieurs paires d'électrodes positionnées de manière normalisée. Le système international 10/20 de placement des électrodes est le système le plus couramment utilisé. Les chiffres 10 et 20 dénotent le fait que la distance entre deux électrodes adjacentes est de 10 % à 20 % de la distance totale soit entre le devant et le derrière de la tête ou entre le côté gauche et le côté droit du crâne. Au total, 21 électrodes sont posées sur le cuir chevelu selon des règles strictes. Il existe également d'autres systèmes de placement des électrodes tels que le système 10/10. Une même électrode de référence peut être commune à plusieurs électrodes actives.

Il est connu que les électrodes EEG ne captent pas seulement le signal souhaité, mais également toutes sortes de pollution électromagnétique. La source de perturbations la plus importante vient des lignes électriques, qui émettent des oscillations électromagnétiques de 50 Hertz (ou 60 Hz). Le signal total capté par une électrode EEG est normalement beaucoup plus élevé que le signal EEG d'intérêt. Dans un montage à deux électrodes tel que décrit ci-dessus, comme les deux électrodes captent à peu près le même bruit, un amplificateur soustrait alors les deux signaux, éliminant ainsi les perturbations de mode commun, ce qui permet de ne conserver en sortie que le signal EEG souhaité. En outre, puisque la perturbation électromagnétique captée par les électrodes est généralement beaucoup plus élevée que le signal EEG d'intérêt, lors de l'analyse du signal EEG, il est également possible de filtrer de façon sélective ces perturbations.

Comme décrit ci-dessus, la différence de potentiel mesurée entre deux électrodes appliquées sur un être vivant est souvent très faible, de l'ordre du millivolt ou du microvolt selon les applications, la nature et la position des électrodes rendant nécessaire une amplification du signal et un filtrage pour améliorer la qualité du signal.

Le principe de l'invention est de proposer une nouvelle architecture d'électrode active. De manière non limitative, celle-ci pourra être employée pour la mesure d'une activité électrique en étant implantée dans le corps d'un être vivant ou simplement positionnée en contact avec la peau de l'être vivant. Elle sera avantageusement associée à une autre électrode pour capter une différence de potentiel.

Dans la suite de la description, on considérera que l'invention peut être mise en œuvre en utilisant des transistors à effet de champ ou bipolaire. Pour généraliser cet aspect, chaque transistor comporte ainsi trois bornes :
- Une première borne formant le drain pour un transistor à effet de champ ou le collecteur d'un transistor bipolaire ;
- Une deuxième borne formant la grille d'un transistor à effet de champ ou la base d'un transistor bipolaire ;
- Une troisième borne formant la source d'un transistor à effet de champ ou l'émetteur d'un transistor bipolaire ;

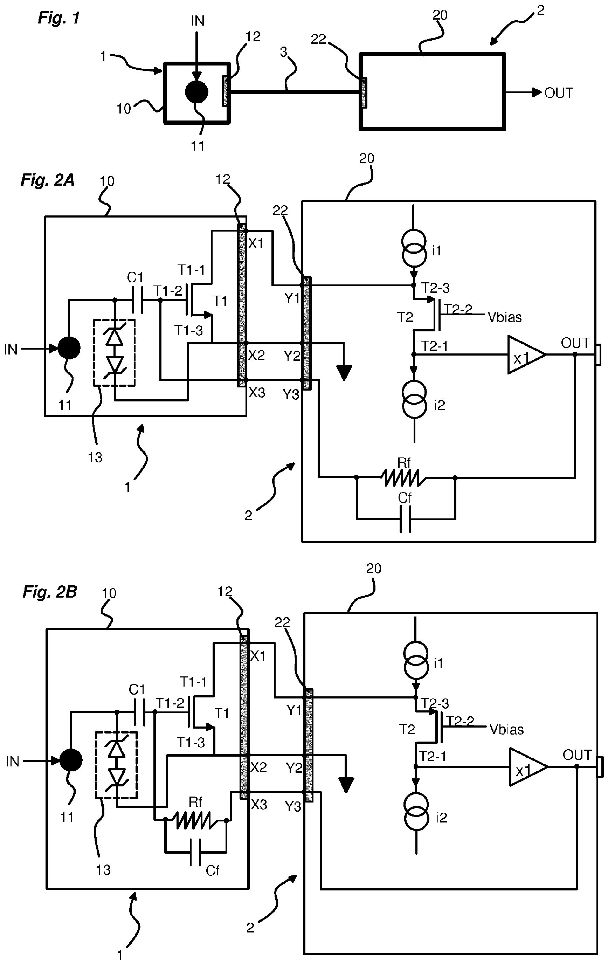

En référence à la figure 1, selon un aspect de l'invention, l'électrode active 1 comporte un boîtier 10 logeant une carte de circuit électronique et un capteur 11 fixé sur ledit boîtier. Le capteur 11 pourra par exemple présenter plusieurs broches ("pins" par exemple montés sur ressort) réalisées dans un matériau électriquement conducteur (par exemple de type Ag, AgCI, Au, Sn...). Le capteur 11 est connecté à la carte électronique logée dans le boîtier et est destiné à capter un signal d'entrée IN.

En référence aux figures 2A et 2B, selon un aspect particulier de l'invention, le circuit électronique de l'électrode active 1 comporte :
- Un transistor T1 à effet de champ ou bipolaire, comprenant trois bornes, dont la deuxième borne est connectée à la borne d'entrée formée par le capteur et destinée à recevoir le signal d'entrée IN ;

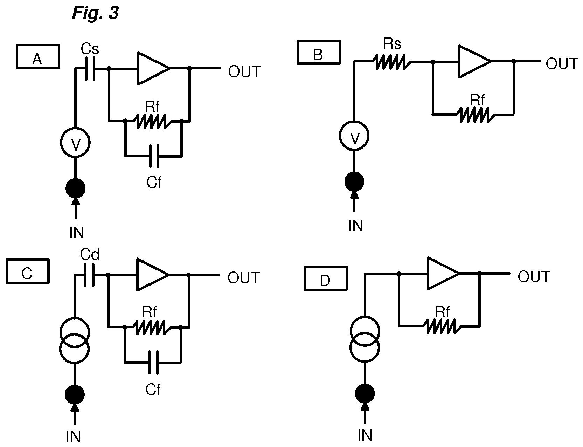

Selon un autre aspect de l'invention, le circuit électronique de l'électrode active 1 pourra comporter un condensateur d'isolement ou de liaison C1. Le choix dépendra notamment du type de transistor T1 employé (à effet de champ ou bipolaire) et du type d'amplification mis en œuvre. Les schémas représentés sur la figure 3 illustrent les différentes configurations possibles.

L'électrode active 1 comporte en outre un dispositif de connexion 12 connecté à sa carte électronique et solidaire du boîtier 10. Ce dispositif de connexion 12 est destiné à relier ladite électrode active 1 à une unité électronique déportée 2. Ce dispositif de connexion 12 comporte au moins trois points de connexion :
- Un premier point de connexion X1 relié à la première borne T1-1 du transistor T1 ;
- Un deuxième point de connexion X2 relié à la troisième borne T1-3 du transistor T1 ;
- Un troisième point de connexion X3 relié uniquement à la deuxième borne T1-2 du transistor T1 ; Par ailleurs, la liaison est directe entre ce troisième point de connexion et la deuxième borne T1-2 du transistor T1.

Le capteur 11 représente ainsi une borne d'entrée destinée à recevoir un signal d'entrée mesuré par l'électrode active.

Le boîtier 10 de l'électrode active n'embarque aucune source d'alimentation, lui permettant notamment d'être implantée dans le corps d'un être vivant, sans rajouter de risque électrique. La polarisation du transistor T1 se fait en effet en courant, par les deux sources de courant i1, i2.

De manière avantageuse, le circuit électronique comporte également un dispositif de protection 13 contre les surtensions, par exemple formé de deux diodes Zener connectées en série par leur anode (en pointillés sur les figures 2A et 2B).

Selon un aspect de l'invention, ladite électrode active 1 est destinée à être connectée, via un câble de liaison 3 ou toute autre solution de liaison électrique, à l'unité électronique déportée 2.

En référence à la figure 2A, cette unité électronique déportée 2 comporte avantageusement un boîtier 20 sur lequel est adapté un dispositif de connexion électrique 22 et qui loge une carte de circuit électronique relié audit dispositif de connexion électrique 22.

Grâce à cette unité électronique déportée, le transistor T1 se trouve être monté en source commune (s'il est à effet de champ) ou à émetteur commun (s'il est bipolaire).

Si le transistor T1 est de type à effet de champ, la sortie est disponible sur le drain et si le transistor T1 est bipolaire, la sortie est sur le collecteur.

Le circuit électronique comporte :
- Un transistor T2 ;
- Une première source de courant i1 connectée à la troisième borne T2-3 du transistor T2 ;
- Une deuxième source de courant i2 connectée à la première borne T2-1 du transistor ;
- Une borne de sortie connectée à la première borne T2-1 du transistor T2 sur laquelle est généré un signal de sortie OUT ;
- Une boucle de retour connectée à la borne de sortie et comprenant au moins une résistance Rf et éventuellement, selon l'architecture, un condensateur Cf connecté en parallèle de ladite résistance ;
- Un amplificateur de gain (x1) connecté à la première borne du transistor T2 et configuré pour baisser l'impédance de sortie du dispositif ;

Selon une variante de réalisation représentée sur la figure 2B, l'ensemble formé au moins de la résistance Rf (et de manière optionnelle du condensateur Cf connecté en parallèle de la résistance Rf) peut être positionné dans le premier boîtier 10 de l'électrode active 1. Cet ensemble est donc connecté d'une part à la deuxième borne T1 - 2 du transistor T1 et d'autre part au troisième point de connexion X3 du dispositif de connexion 12 de l'électrode active 1. Cette architecture présente notamment l'avantage de pouvoir rendre le nœud de masse virtuel formé de la deuxième borne T1-2 du transistor T1 le plus petit possible.

Le transistor T2 est polarisé par les sources de courant i1, i2 et par l'application d'une tension de polarisation (Vbias) sur sa deuxième borne de commande.

Le transistor T1 sera avantageusement à effet de champ et le deuxième transistor T2 de type bipolaire. Préférentiellement, on pourra notamment opter pour une combinaison comprenant un JFET pour le transistor T1 et un transistor T2 bipolaire de type PNP ou NPN, selon la configuration du JFET.

Dans cette unité électronique déportée 2, le dispositif de connexion électrique 22 comporte trois points de connexion :
- Un premier point de connexion Y1 relié à la troisième borne du transistor T2 ;
- Un deuxième point de connexion Y2 relié à la masse ;
- Un troisième point de connexion Y3 ;

Dans l'architecture de la figure 2A, le troisième point de connexion Y3 est relié à la boucle de retour qui est connectée à la borne de sortie.

Dans l'architecture de la figure 2B, le troisième point de connexion Y3 est relié directement à la borne de sortie, la boucle de retour étant alors dans le circuit de l'électrode active 1 et logé dans son boîtier 10.

De manière avantageuse, chaque source de courant est une source active de courant, par exemple de type "Widlar".

Selon un aspect particulier de l'invention, l'électrode active 1 est destinée à être connectée à l'unité électronique déportée 2 par le câble de liaison 3. Ce câble de liaison permet de relier :
- Le premier point de connexion X1 du dispositif de connexion de l'électrode active au premier point de connexion Y1 du dispositif de connexion de l'unité électronique déportée ;
- Le deuxième point de connexion X2 du dispositif de connexion de l'électrode active au deuxième point de connexion Y2 du dispositif de connexion de l'unité électronique déportée ;
- Le troisième point de connexion X3 du dispositif de connexion de l'électrode active au troisième point de connexion Y3 du dispositif de connexion de l'unité électronique déportée ;

Une quatrième liaison pourrait également être employée pour relier une quatrième borne du dispositif de connexion de l'électrode active à une quatrième borne du dispositif de connexion de l'unité électronique déportée. Cette quatrième liaison permettrait de créer un écran électromagnétique de type cage de Faraday. Cette connexion permet de relier les boîtiers 10 et 20 entre eux et le potentiel électrique appliqué est celui de l'électrode de masse de la batterie. Les autres éléments du dispositif sont par ailleurs toujours isolés des boîtiers.

Selon un aspect de l'invention, lorsque l'électrode active 1 de l'invention est connectée sur l'unité électronique déportée 2, via le câble de liaison 3, le signal électrique IN appliqué sur la borne d'entrée de l'électrode active 1 est amplifié, permettant d'obtenir un signal électrique OUT sur la borne de sortie.

Selon un aspect avantageux de l'invention, la scission de la structure de l'amplificateur permet de rendre l'électrode active, tout en ne nécessitant pas de source de tension d'alimentation au sein de la partie proche électrode (c'est-à-dire dans le boîtier 10), et tout en fournissant une amplification proche de l'électrode.

De plus, l'impédance de sortie de T1 est faible puisque le Drain de T1 est connecté à l'émetteur de T2, ce qui permet d'éviter la dégradation du signal. Les éléments contenus dans le boitier 10 pour les configurations des figures 2A et 2B peuvent être facilement intégrés ensemble au plus proche d'une électrode de contact passive. Les éléments contenus dans le boîtier 20 permettent de compléter la structure de l'amplificateur et peuvent être déportés. L'unité électronique complète permet alors de réaliser une électrode active et de fournir un signal de sortie OUT, à la fois amplifié et sous basse impédance.

Comme précisé ci-dessus, le signal d'entrée IN appliqué sur l'électrode active est souvent très faible (c'est le cas en EEG). La connexion de l'unité électronique déportée 2 sur l'électrode active permet d'assurer un signal en entrée de l'unité électronique peu perturbée, même après avoir transité sur le câble.

Par ailleurs, la solution de l'invention en deux parties distinctes permet d'éliminer toute source d'alimentation de l'électrode active 1 en la plaçant dans l'unité électronique déportée 2, permettant ainsi de l'implanter dans le corps d'un être vivant en minimisant les risques pour l'être vivant.

Les schémas A à D présentés sur la figure 3 permettent d'illustrer différentes architectures électroniques réalisables dans le cadre de l'invention, selon que l'amplificateur est à condensateurs ou à résistances.
- Schéma A : En présence d'une source de tension V connectée au capteur 11, association d'un condensateur de liaison Cs en entrée et d'un filtre formé d'une résistance Rf et d'un condensateur Cf en parallèle sur la boucle de retour ;
- Schéma B : En présence d'une source de tension V connectée au capteur 11, association d'une résistance de liaison Rs en entrée et d'un filtre formé d'une résistance Rf sur la boucle de retour ;
- Schéma C : En présence d'une source de courant I connectée au capteur 11, association d'un condensateur de découplage Cd en entrée et d'un filtre formé d'une résistance Rf et d'un condensateur Cf en parallèle sur la boucle de retour ;
- Schéma D : En présence d'une source de courant I connectée au capteur 11, utilisation d'un filtre formé d'une résistance Rf sur la boucle de retour ;

## Revendications

1. Electrode active (1) de mesure d'activité électrique implantable ou non-implantable, **caractérisée en ce qu'**elle comporte :
- Un boîtier (10) ;
- Un capteur (11) de mesure adapté sur ledit premier boîtier et destiné à capter un signal électrique d'entrée (IN) ;
- Ledit boîtier (10) logeant un circuit électronique comprenant un transistor (T1) qui comporte une première borne, une deuxième borne reliée audit capteur et formant une entrée et une troisième borne ;
- Un dispositif de connexion électrique (12) comprenant un premier point de connexion (X1) relié à ladite première borne du transistor (T1), un deuxième point de connexion (X2) relié à la troisième borne du transistor (T1) et un troisième point de connexion (X3) relié uniquement à la deuxième borne du transistor (T1).

2. Electrode active selon la revendication 1, **caractérisée en ce que** le circuit électronique comporte un condensateur (C1) connecté au capteur et à la deuxième borne du transistor.

3. Electrode active selon la revendication 1, **caractérisée en ce que** le circuit électronique comporte un dispositif de protection (13) connecté au capteur et à la deuxième borne du transistor.

4. Electrode active selon l'une des revendications 1 à 3, **caractérisée en ce que** le transistor (T1) est choisi parmi un type bipolaire ou à effet de champ.

5. Electrode active selon l'une des revendications 1 à 4, **caractérisée en ce que** le circuit électronique comporte un ensemble comprenant au moins une résistance (Rf) et un condensateur (Cf) connecté en parallèle de ladite résistance, connecté d'une part à la deuxième borne (T1-2) du transistor (T1) et d'autre part au troisième point de connexion (X3).

6. Dispositif de mesure d'activité électrique, **caractérisé en ce qu'**il comporte :
- Une électrode active (1) telle que définie dans l'une des revendications 1 à 4;
- Une unité électronique déportée (2) comprenant un deuxième boîtier logeant un circuit électronique qui comporte :
∘ Un transistor (T2) comprenant une première borne, une deuxième borne et une troisième borne ;
∘ Une première source de courant (i1) connectée à la troisième borne du transistor ;
∘ Une deuxième source de courant (i2) connectée à la première borne du transistor ;
∘ Une borne de sortie connectée à la première borne du transistor (T2) ;
∘ Une boucle de retour connectée à la borne de sortie et comprenant au moins une résistance (Rf) et un condensateur (Cf) connecté en parallèle de ladite résistance (Rf) ;
- Ladite unité électronique déportée (2) comprenant un dispositif de connexion électrique (22) comprenant :
∘ Un premier point de connexion (Y1) relié à la troisième borne du transistor (T2) ;
∘ Un deuxième point de connexion (Y2) relié à la masse ;
∘ Un troisième point de connexion (Y3) auquel est reliée la boucle de retour ;
- Un câble reliant l'électrode active à l'unité électronique déportée de sorte que :
∘ Le premier point de connexion (X1) du dispositif de connexion (12) de l'électrode active (1) est relié au premier point de connexion (Y1) du dispositif de connexion (22) de l'unité électronique déportée ;
∘ Le deuxième point de connexion (X2) du dispositif de connexion de l'électrode active est relié au deuxième point de connexion (Y2) du dispositif de connexion (22) de l'unité électronique déportée ;
∘ Le troisième point de connexion (X3) du dispositif de connexion de l'électrode active est relié au troisième point de connexion (Y3) du dispositif de connexion (22) de l'unité électronique déportée ;

7. Dispositif de mesure d'activité électrique, **caractérisé en ce qu'**il comporte :
- Une électrode active (1) telle que définie dans la revendication 5,
- Une unité électronique déportée (2) comprenant un deuxième boîtier logeant un circuit électronique qui comporte :
∘ Un transistor (T2) comprenant une première borne, une deuxième borne et une troisième borne ;
∘ Une première source de courant (i1) connectée à la troisième borne du transistor ;
∘ Une deuxième source de courant (i2) connectée à la première borne du transistor ;
∘ Une borne de sortie connectée à la première borne du transistor (T2) ;
- Ladite unité électronique déportée (2) comprenant un dispositif de connexion électrique (22) comprenant :
∘ Un premier point de connexion (Y1) relié à la troisième borne du transistor (T2) ;
∘ Un deuxième point de connexion (Y2) relié à la masse ;
∘ Un troisième point de connexion (Y3) relié à la borne de sortie ;
- Un câble reliant l'électrode active à l'unité électronique déportée de sorte que :
∘ Le premier point de connexion (X1) du dispositif de connexion (12) de l'électrode active (1) est relié au premier point de connexion (Y1) du dispositif de connexion (22) de l'unité électronique déportée ;
∘ Le deuxième point de connexion (X2) du dispositif de connexion de l'électrode active est relié au deuxième point de connexion (Y2) du dispositif de connexion (22) de l'unité électronique déportée ;
∘ Le troisième point de connexion (X3) du dispositif de connexion de l'électrode active est relié au troisième point de connexion (Y3) du dispositif de connexion (22) de l'unité électronique déportée ;

8. Dispositif selon la revendication 6 ou 7, **caractérisé en ce que** chaque transistor (T1, T2) est choisi parmi un type bipolaire ou à effet de champ.

## Patentansprüche

1. Implantierbare oder nicht implantierbare aktive Elektrode (1) zum Messen einer elektrischen Aktivität, **dadurch gekennzeichnet, dass** sie Folgendes umfasst:
- ein Gehäuse (10);
- einen Messfühler (11), der an dem ersten Gehäuse angebracht ist und dazu bestimmt ist, ein elektrisches Eingangssignal (IN) zu erfassen;
- wobei das Gehäuse (10) eine elektronische Schaltung aufnimmt, die einen Transistor (T1) beinhaltet, der einen ersten Anschluss, einen zweiten Anschluss, der mit dem Fühler verbunden ist und einen Eingang bildet, und einen dritten Anschluss umfasst;
- eine elektrische Verbindungsvorrichtung (12), die einen ersten Verbindungspunkt (X1), der mit dem ersten Anschluss des Transistors (T1) verbunden ist, einen zweiten Verbindungspunkt (X2), der mit dem dritten Anschluss des Transistors (T1) verbunden ist, und einen dritten Verbindungspunkt (X3), der nur mit dem zweiten Anschluss des Transistors (T1) verbunden ist, beinhaltet.

2. Aktive Elektrode nach Anspruch 1, **dadurch gekennzeichnet, dass** die elektronische Schaltung einen Kondensator (C1), der mit dem Fühler und dem zweiten Anschluss des Transistors verbunden ist, umfasst.

3. Aktive Elektrode nach Anspruch 1, **dadurch gekennzeichnet, dass** die elektronische Schaltung eine Schutzvorrichtung (13), die mit dem Fühler und dem zweiten Anschluss des Transistors verbunden ist, umfasst.

4. Aktive Elektrode nach einem der Ansprüche 1 bis 3, **dadurch gekennzeichnet, dass** der Transistor (T1) aus einem Bipolartransistor oder einem Feldeffekttransistor ausgewählt ist.

5. Aktive Elektrode nach einem der Ansprüche 1 bis 4, **dadurch gekennzeichnet, dass** die elektronische Schaltung eine Anordnung, die mindestens einen Widerstand (Rf) und einen zu dem Widerstand parallel geschalteten Kondensator (Cf) beinhaltet, umfasst, die einerseits mit dem zweiten Anschluss (T1-2) des Transistors (T1) und andererseits mit dem dritten Verbindungspunkt (X3) verbunden ist.

6. Vorrichtung zum Messen einer elektrischen Aktivität, **dadurch gekennzeichnet, dass** sie Folgendes umfasst:
- eine aktive Elektrode (1) wie in einem der Ansprüche 1 bis 4 definiert;
- eine abgesetzt angeordnete elektronische Einheit (2), die ein zweites Gehäuse beinhaltet, das eine elektronische Schaltung aufnimmt, die Folgendes umfasst:
∘ einen Transistor (T2), der einen ersten Anschluss, einen zweiten Anschluss und einen dritten Anschluss beinhaltet;
∘ eine erste Stromquelle (i1), die mit dem dritten Anschluss des Transistors verbunden ist;
∘ eine zweite Stromquelle (i2), die mit dem ersten Anschluss des Transistors verbunden ist;
∘ einen Ausgangsanschluss, der mit dem ersten Anschluss des Transistors (T2) verbunden ist;
∘ eine Rückkopplungsschleife, die mit dem Ausgangsanschluss verbunden ist und mindestens einen Widerstand (Rf) und einen zu dem Widerstand (Rf) parallel geschalteten Kondensator (Cf) beinhaltet;
- wobei die abgesetzt angeordnete elektronische Einheit (2) eine elektrische Verbindungsvorrichtung (22) beinhaltet, die Folgendes beinhaltet:
∘ einen ersten Verbindungspunkt (Y1), der mit dem dritten Anschluss des Transistors (T2) verbunden ist;
∘ einen zweiten Verbindungspunkt (Y2), der mit Masse verbunden ist;
∘ einen dritten Verbindungspunkt (Y3), mit dem die Rückkopplungsschleife verbunden ist;
- ein Kabel, das die aktive Elektrode mit der abgesetzt angeordneten elektronischen Einheit verbindet, sodass:
∘ der erste Verbindungspunkt (X1) der Verbindungsvorrichtung (12) der aktiven Elektrode (1) mit dem ersten Verbindungspunkt (Y1) der Verbindungsvorrichtung (22) der abgesetzt angeordneten elektronischen Einheit verbunden ist;
∘ der zweite Verbindungspunkt (X2) der Verbindungsvorrichtung der aktiven Elektrode mit dem zweiten Verbindungspunkt (Y2) der Verbindungsvorrichtung (22) der abgesetzt angeordneten elektronischen Einheit verbunden ist;
∘ der dritte Verbindungspunkt (X3) der Verbindungsvorrichtung der aktiven Elektrode mit dem dritten Verbindungspunkt (Y3) der Verbindungsvorrichtung (22) der abgesetzt angeordneten elektronischen Einheit verbunden ist.

7. Vorrichtung zum Messen einer elektrischen Aktivität, **dadurch gekennzeichnet, dass** sie Folgendes umfasst:
- eine aktive Elektrode (1) wie in Anspruch 5 definiert,
- eine abgesetzt angeordnete elektronische Einheit (2), die ein zweites Gehäuse beinhaltet, das eine elektronische Schaltung aufnimmt, die Folgendes umfasst:
∘ einen Transistor (T2), der einen ersten Anschluss, einen zweiten Anschluss und einen dritten Anschluss beinhaltet;
∘ eine erste Stromquelle (i1), die mit dem dritten Anschluss des Transistors verbunden ist;
∘ eine zweite Stromquelle (i2), die mit dem ersten Anschluss des Transistors verbunden ist;
∘ einen Ausgangsanschluss, der mit dem ersten Anschluss des Transistors (T2) verbunden ist;
- wobei die abgesetzt angeordnete elektronische Einheit (2) eine elektrische Verbindungsvorrichtung (22) beinhaltet, die Folgendes beinhaltet:
∘ einen ersten Verbindungspunkt (Y1), der mit dem dritten Anschluss des Transistors (T2) verbunden ist;
∘ einen zweiten Verbindungspunkt (Y2), der mit Masse verbunden ist;
∘ einen dritten Verbindungspunkt (Y3), der mit dem Ausgangsanschluss verbunden ist;
- ein Kabel, das die aktive Elektrode mit der abgesetzt angeordneten elektronischen Einheit verbindet, sodass:
∘ der erste Verbindungspunkt (X1) der Verbindungsvorrichtung (12) der aktiven Elektrode (1) mit dem ersten Verbindungspunkt (Y1) der Verbindungsvorrichtung (22) der abgesetzt angeordneten elektronischen Einheit verbunden ist;
∘ der zweite Verbindungspunkt (X2) der Verbindungsvorrichtung der aktiven Elektrode mit dem zweiten Verbindungspunkt (Y2) der Verbindungsvorrichtung (22) der abgesetzt angeordneten elektronischen Einheit verbunden ist;
∘ der dritte Verbindungspunkt (X3) der Verbindungsvorrichtung der aktiven Elektrode mit dem dritten Verbindungspunkt (Y3) der Verbindungsvorrichtung (22) der abgesetzt angeordneten elektronischen Einheit verbunden ist.

8. Vorrichtung nach Anspruch 6 oder 7, **dadurch gekennzeichnet, dass** jeder Transistor (T1, T2) aus einem Bipolartransistor oder einem Feldeffekttransistor ausgewählt ist.

## Claims

1. Implantable or non-implantable active electrode (1) for measuring electrical activity, **characterized in that** it comprises:
- a casing (10);
- a suitable measurement sensor (11) on said first casing and intended to sense an input electrical signal (IN);
- said casing (10) housing an electronic circuit comprising a transistor (T1) that comprises a first terminal, a second terminal that is connected to said sensor and that forms an input and a third terminal;
- an electrically connecting device (12) comprising a first connection point (X1) connected to said first terminal of the transistor (T1), a second connection point (X2) connected to the third terminal of the transistor (T1) and a third connection point (X3) connected solely to the second terminal of the transistor (T1).

2. Active electrode according to Claim 1, **characterized in that** the electronic circuit comprises a capacitor (C1) connected to the sensor and to the second terminal of the transistor.

3. Active electrode according to Claim 1, **characterized in that** the electronic circuit comprises a protective device (13) connected to the sensor and to the second terminal of the transistor.

4. Active electrode according to one of Claims 1 to 3, **characterized in that** the transistor (T1) is chosen from a bipolar transistor and a field-effect transistor.

5. Active electrode according to one of Claims 1 to 4, **characterized in that** the electronic circuit comprises an assembly comprising at least a resistor (Rf) and a capacitor (Cf) connected in parallel with said resistor, connected on the one hand to the second terminal (T1-2) of the transistor (T1) and on the other hand to the third connection point (X3).

6. Device for measuring electrical activity, **characterized in that** it comprises:
- An active electrode (1) such as defined in one of Claims 1 to 4;
- A remote electronic unit (2) comprising a second casing housing an electronic circuit that comprises:
∘ a transistor (T2) comprising a first terminal, a second terminal and a third terminal;
∘ a first current source (i1) connected to the third terminal of the transistor;
∘ a second current source (i2) connected to the first terminal of the transistor;
∘ an output terminal connected to the first terminal of the transistor (T2);
∘ a return loop connected to the output terminal and comprising at least a resistor (Rf) and a capacitor (Cf) connected in parallel with said resistor (Rf);
- said remote electronic unit (2) comprising an electrically connecting device (22) comprising
∘ a first connection point (Y1) connected to the third terminal of the transistor (T2);
∘ a second connection point (Y2) connected to ground;
∘ a third connection point (Y3) to which the return loop is connected;
- a cable connecting the active electrode to the remote electronic unit so that:
∘ the first connection point (X1) of the connecting device (12) of the active electrode (1) is connected to the first connection point (Y1) of the connecting device (22) of the remote electronic unit;
∘ the second connection point (X2) of the connecting device of the active electrode is connected to the second connection point (Y2) of the connecting device (22) of the remote electronic unit;
∘ the third connection point (X3) of the connecting device of the active electrode is connected to the third connection point (Y3) of the connecting device (22) of the remote electronic unit.

7. Device for measuring electrical activity, **characterized in that** it comprises:
- an active electrode (1) such as defined in Claim 5,
- a remote electronic unit (2) comprising a second casing housing an electronic unit that comprises:
∘ a transistor (T2) comprising a first terminal, a second terminal and a third terminal;
∘ a first current source (i1) connected to the third terminal of the transistor;
∘ a second current source (i2) connected to the first terminal of the transistor;
∘ an output terminal connected to the first terminal of the transistor (T2);
- said remote electronic unit (2) comprising an electrically connecting device (22) comprising
∘ a first connection point (Y1) connected to the third terminal of the transistor (T2);
∘ a second connection point (Y2) connected to ground;
∘ a third connection point (Y3) connected to the output terminal;
- a cable connecting the active electrode to the remote electronic unit so that:
∘ the first connection point (X1) of the connecting device (12) of the active electrode (1) is connected to the first connection point (Y1) of the connecting device (22) of the remote electronic unit;
∘ the second connection point (X2) of the connecting device of the active electrode is connected to the second connection point (Y2) of the connecting device (22) of the remote electronic unit;
∘ the third connection point (X3) of the connecting device of the active electrode is connected to the third connection point (Y3) of the connecting device (22) of the remote electronic unit.

8. Device according to Claim 6 or 7, **characterized in that** each transistor (T1, T2) is chosen from a bipolar transistor and a field-effect transistor.
